# EUROPÄISCHE PATENTSCHRIFT

(11) **EP 1 592 467 B1**
(45) Veröffentlichungstag und Bekanntmachung des Hinweises auf die Patenterteilung: **25.12.2013**
(21) Anmeldenummer: 04708297.9
(22) Anmeldetag: 05.02.2004
(51) Int. Cl.: A61M 1/06

(54) **Portable Brustpumpe**
Portable breast pump
Pompe tire-lait portable

(30) Priorität: 10.02.2003 CH 197032003
(43) Veröffentlichungstag der Anmeldung: 09.11.2005
(73) Patentinhaber: Medela Holding AG, 6340 Baar (CH)
(72) Erfinder: PFENNIGER, Erich, CH-6030 Ebikon (CH); WEBER, Beda, CH-5643 Sins (CH); RÖLLIN, Richard, CH-6313 Menzingen (CH); KERSCHDORFER, Markus, CH-5643 Sins (CH)
(74) Vertreter: Clerc, Natalia
(86) Internationale Anmeldenummer: PCT/CH2004/000061
(87) Internationale Veröffentlichungsnummer: WO 2004/069306

(56) Entgegenhaltungen:
- EP-A- 1 088 569
- GB-A- 2 366 732
- US-A- 5 472 317
- US-A- 5 571 084
- US-A1- 2001 047 148
- US-B2- 6 379 327

## Beschreibung

### Technisches Gebiet

Die Erfindung betrifft eine portable Brustpumpe sowie eine Saugeinheit zur Verwendung mit dieser portablen Brustpumpe gemäss Oberbegriff des Patentanspruchs 1 beziehungsweise 2.

### Stand der Technik

Portable Brustpumpen sind bekannt. Sie weisen den Vorteil auf, dass eine Mutter sie überall mitnehmen und somit auch während den Stillmonaten eine gewisse Unabhängigkeit bewahren kann. Nun ist es jedoch nicht immer einfach, einen geeigneten Raum zu finden, in welcher die Mutter ungestört die Milch abpumpen kann. Insbesondere berufstätige Mütter müssen dies oft in der Damentoilette am Arbeitsort durch-führen. In derartigen Räumen ist es jedoch meistens schwierig, eine geeignete Abstellfläche für die Brustpumpe zu finden.

US-A-6'379'327 offenbart ein Brustpumpensystem, bei welchem die Mutter alle Elemente der Brustpumpe am Körper tragen kann. Dieses System umfasst einen Büstenhalter mit einer integrierten Brustaufsatzhaube sowie ein breites um den Bauch getragenes Band mit einer ersten Tasche zur Aufnahme einer Saugeinheit und einer zweiten Tasche zur Aufnahme eines Milchaufnahmebehälters. Die Saugeinheit und der Milchaufnahmebehälter sind jeweils über eine eigene Leitung mit der Brustaufsatzhaube verbunden. Das gesamte System wird unter der Kleidung getragen. Dies weist den Nachteil auf, dass die Mutter die Brustpumpe stets mit sich herumtragen muss, was als unangenehm empfunden wird und die Mutter in ihrer übrigen Tätigkeit behindert. Zudem weist die Saugeinheit ein nicht unwesentliches Gewicht auf, so dass die Mutter sie ungern über längere Zeit tragen wird. Des weiteren sind die Bedienungsknöpfe der Brustpumpe ebenfalls unter der Kleidung versteckt und somit nicht gut zugänglich.

Eine ähnliche Brustpumpe ist auch in US-A-6'440'100 dargestellt, wobei hier die Anordnung der Saugeinheit nicht näher erläutert ist.

GB-A-2'366'732 beschreibt eine portable Brustpumpe, welche an einem Bügel über der Schulter getragen wird, wobei der Bügel längenverstellbar ist. Die Saugeinheit ist am unteren vorderen Ende des Bügels angeordnet. An der Saugeinheit sind über ein Kopplungsteil die Brustaufsatzhaube und der Milchaufnahmebehälter befestigt. Dieses System lässt sich zwar über der Kleidung tragen und muss somit nur bei Bedarf angehängt werden. Nachteilig ist jedoch, dass es relativ starr ausgebildet ist, so dass sich die Mutter während dem Abpumpen der Milch kaum bewegen kann. Zudem muss die Saugeinheit relativ klein ausgebildet werden, damit der Bügel nicht zu weit vorsteht. Kleine Saugeinheiten weisen jedoch üblicherweise auch eine geringe Saugleistung auf und/oder verfügen lediglich über eine rudimentäre Steuerung.

### Darstellung der Erfindung

Es ist deshalb Aufgabe der Erfindung, eine portable Brustpumpe der eingangs genannten Art zu schaffen, welche die obengenannten Nachteile behebt.

Diese Aufgabe löst eine Brustpumpe sowie eine Saugeinheit zur Verwendung mit dieser Brustpumpe mit den Merkmalen des Patentanspruchs 1 beziehungsweise 2.

Die erfindungsgemässe Brustpumpe weist eine Saugeinheit und eine Auffangeinheit auf, welche mittels einer flexiblen Saugleitung miteinander verbunden sind. Die Saugeinheit verfügt über ein Gehäuse, welches mit mindestens einem Befestigungsmittel versehen ist. Das Befestigungsmittel dient zur Befestigung des Gehäuses an einem Tragelement, welches am Körper der Mutter befestigbar ist.

Vorzugsweise ist die Verbindung zwischen Tragelement und Befestigungsmittel lösbar. Vorzugsweise lässt sich das Gehäuse an einem Hosen- oder Kleidgürtel der Mutter befestigen oder an einer Schlaufe um den Hals tragen.

Diese Brustpumpe mit ihrer Saugeinheit erlaubt es der Mutter, sich auch während dem Abpumpen der Milch frei zu bewegen. Zudem kann die Mutter wählen, an welcher Stelle beziehungsweise wie sie die Saugeinheit tragen möchte.

Da das Gehäuse flach ausgebildet ist, erhöht sich der Tragkomfort für die Mutter. Zudem wird verhindert, dass sie mit dem Gehäuse irgendwo hängen bleibt. Vorteilhaft ist ferner, dass sich das Gehäuse in seiner Form relativ frei gestalten lässt, so dass es als modisches Accessoire getragen werden kann, ohne auf den ersten Blick als Saugeinheit einer Brustpumpe erkannt zu werden. Es kann zudem so ausgebildet sein, dass es einen genügenden Tragkomfort aufweist. Zudem kann die Saugeinheit genügend gross ausgebildet werden, um eine genügend grosse elektrische Speicherzelle sowie die gewünschte Elektronik aufzunehmen.

Da die Saugeinheit offen getragen wird, sind allfällige Bedienungstasten gut zugänglich. Um die Zugänglichkeit noch zu erhöhen sind sie vorzugsweise in einer Vorderseite des Gehäuses angeordnet.

Weitere vorteilhafte Ausführungsformen gehen aus den abhängigen Patentansprüchen hervor.

### Kurze Beschreibung der Zeichnung

Im folgenden wird der Erfindungsgegenstand anhand eines bevorzugten Ausführungsbeispiels, welches in der beiliegenden Zeichnung dargestellt ist, erläutert. Es zeigen:
- Figur 1: eine erfindungsgemässe portable Brustpumpe mit einer Saugeinheit in einer ersten Tragvariante;
- Figur 2: die Brustpumpe gemäss Figur 1 in einer zweiten Tragvariante und
- Figur 3: die Saugeinheit gemäss Figur 1 in einer Ansicht von hinten.

### Wege zur Ausführung der Erfindung

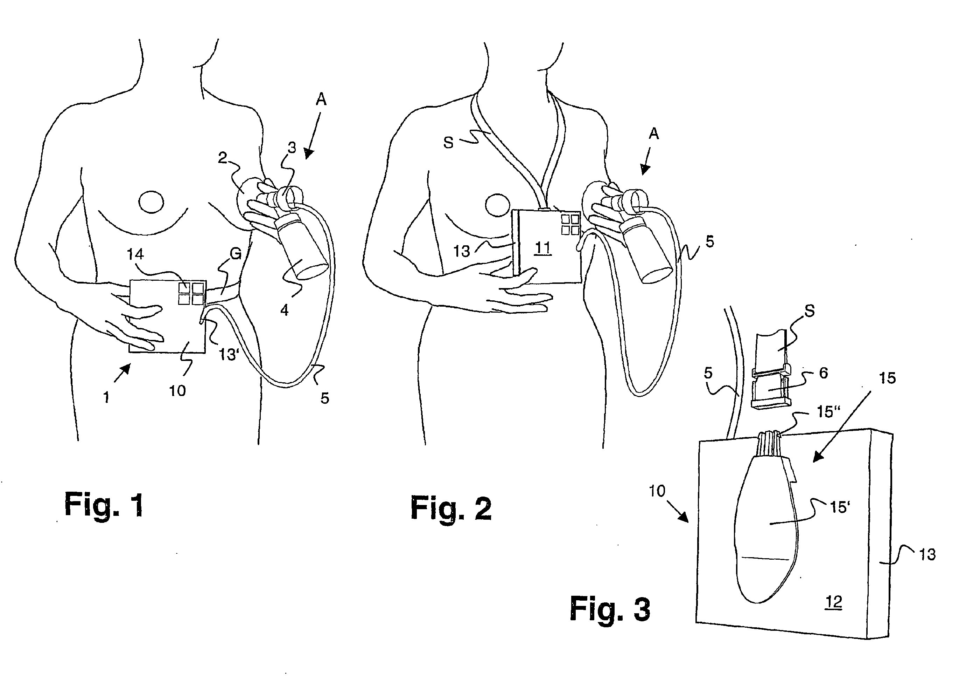

In Figur 1 ist eine erfindungsgemässe portable Brustpumpe dargestellt. Die Brustpumpe weist eine Auffangeinheit A und eine damit über eine Saugleitung 5 verbundene Saugeinheit 1 auf. Die Saugleitung 5 ist üblicherweise ein flexibler Kunststoffschlauch.

Die Auffangeinheit A besteht in diesem bevorzugten Beispiel aus einer Brustaufsatzhaube 2, einem Kopplungselement 3 und einem Milchaufnahmebehälter 4. Das Kopplungselement 3 verbindet die Brustaufsatzhaube 2 mit dem Milchaufnahmebehälter 4 zu einer starren Einheit, welche an die Mutterbrust angesetzt und während dem Absaugen der Milch in der Hand gehalten werden kann. Es ist jedoch möglich, auch die Verbindung zwischen Milchaufnahmebehälter 4 und Brustaufsatzhaube 2 flexibel zu gestalten und diese mittels eines weiteren Verbindungsschlauchs miteinander zu verbinden. So kann die Brustaufsatzhaube 2 an die Brust gehalten werden oder sie kann in einen Büstenhalter integriert sein. In dieser Variante kann der Behälter 4 mit einem Befestigungsmittel versehen sein, um ihn beispielsweise an einem Gürtel anzuhängen. Er kann aber auch in einer Hand gehalten oder auf eine Abstellfläche gestellt werden. Die Ausführungsform mit der starren Einheit ist jedoch bezüglich Mobilität und Handhabung die bevorzugte Lösung.

Die Saugeinheit 1 umfasst die im Stand der Technik bekannten Elemente zur Erzeugung eines für das Abpumpen der Milch genügenden Unterdrucks in der Brustaufsatzhaube 2. Diese Elemente umfassen eine nicht dargestellte Saugpumpe, eine Steuerelektronik sowie ein elektrisches Speicherelement, beispielsweise eine Batterie. Alle diese Elemente sind in einem gemeinsamen Gehäuse 10 angeordnet. Dieses besteht vorzugsweise aus Kunststoff und ist starr ausgebildet. Es weist entweder Anschlussöffnungen für eine Steckverbindung zur Verbindung mit einem elektrischen Ladegerät für das Speicherelement auf oder es kann mindestens im Bereich der Batterie geöffnet werden, um die Batterie auszuwechseln.

Das Gehäuse 10 ist flach ausgebildet, mit einer Vorderseite 11, einer Rückseite 12 und einer diese verbindenden Mantelfläche 13. Der Querschnitt des Gehäuses 10 kann beliebig gewählt werden. Am Gehäuse 10, beispielsweise in der Mantelfläche 13, ist eine Ausnehmung 13' vorhanden zur Aufnahme eines Endes der Saugleitung 5. Diese Saugleitung 5 ist vorzugsweise lösbar in diese Ausnehmung 13' einsteckbar.

Am Gehäuse 10 ist mindestens ein Bedienungsmittel 14 zur Betätigung der Saugpumpe vorhanden. In diesem Beispiel sind dies Tasten, welche in der Vorderseite 11 des Gehäuses 10 eingelassen sind.

Am Gehäuse 10 ist ferner ein Befestigungsmittel 15 angeordnet, um das Gehäuse 10 an einem Tragelement zu befestigen. Das Tragelement ist in diesem Beispiel ein Gürtel G. Das Befestigungsmittel 15 ist vorzugsweise an der dem Bedienungsmittel 15 gegenüberliegenden Seite des Gehäuses 10 angeordnet, hier somit auf der Rückseite 12, wie dies in Figur 3 erkennbar ist. Das Befestigungsmittel 15 besteht in diesem Beispiel aus einer federnden Klammer 15', insbesondere aus Metall oder Kunststoff, und einem Steckverschlussteil 15", hier aus Steckstiften 150, welche teilweise mit Rückhaltehaken 151 versehen sind. Es ist jedoch auch möglich, das Gehäuse 10 nur mit der Klammer 15' oder nur mit dem Steckverschlussteil 15" zu versehen. Zudem ist es möglich, andere bekannte Befestigungsmittel, beispielsweise einen Klettverschluss, zu verwenden.

Die erfindungsgemässe Saugeinheit 1 lässt sich mittels der Klammer 15' auf einfache Art und Weise an einem Gürtel G einhaken. Dies muss nicht ein spezieller Gürtel sein, sondern es kann sich um einen von der Mutter individuell ausgewählten Gürtel handeln.

In der Ausführungsform gemäss Figur 2 wird die Saugeinheit 1 mit einer Schlaufe S um den Hals getragen. Die Schlaufe S kann entweder fest mit dem Gehäuse 10 verbunden sein. Vorzugsweise ist aber eine lösbare Verbindung gewählt. Beispielsweise lässt sich das untere Ende der Schlaufe S zur Verbindung mit dem Steckverschlussteil 15" mit einem zugehörigen Steckverschlussteil 6 versehen, wie es in Figur 3 dargestellt ist.

Die Saugeinheit 1 lässt sich jedoch auch auf andere Art und Weise tragen, beispielsweise am Arm oder um die Schulter gehängt.

Die erfindungsgemässe Brustpumpe ist einfach zu bedienen, erlaubt ein Absaugen der Milch auch in beengten Räumen ohne Abstellflächen und behindert zudem die Mutter auch während dem Abpumpen in ihrer Mobilität nicht.

### Bezugszeichenliste

- G: Gürtel
- S: Schlaufe
- A: Auffangeinheit
- 1: Saugeinheit
- 10: Gehäuse
- 11: Vorderseite
- 12: Rückseite
- 13: Mantelfläche
- 13': Ausnehmung
- 14: Bedienungsmittel
- 15: Befestigungsmittel
- 15': Klammer
- 15": Steckverschlussteil
- 150: Steckstift
- 151: Rückhaltehaken
- 2: Brustaufsatzhaube
- 3: Kopplungselement
- 4: Milchaufnahmebehälter
- 5: Saugleitung
- 6: Steckverschlussteil

## Patentansprüche

1. Portable Brustpumpe, welche eine Saugeinheit (1), eine Auffangeinheit (A) und eine Saugleitung (5) aufweist, wobei die Saugleitung (5) die Auffangeinheit (A) mit der Saugeinheit (1) verbindet, wobei die Auffangeinheit (A) eine Brustaufsatzhaube (2), einen Milchaufnahmebehälter (4) und eine die Brustaufsatzhaube (2) mit dem Milchaufnahmebehälter (4) verbindendes Kopplungselement (3) aufweist und wobei die Saugeinheit (1) ein Gehäuse (10) aufweist, wobei das Gehäuse (10) mit einem Befestigungsmittel (15) versehen ist zur Befestigung des Gehäuses (10) an einem Tragmittel (G, S), welches am Körper einer Mutter befestigbar ist, wobei das Gehäuse (10) eine Vorderseite (11), eine Rückseite (12) sowie eine die zwei Seiten (11, 12) verbindende Mantelfläche (13) aufweist und wobei das Gehäuse (10) flach ausgebildet ist, in dem Sinne, dass die Vorderseite (11) und die Rückseite (12) breiter ausgebildet sind als die Mantelfläche (13), **dadurch gekennzeichnet, dass** in der Mantelfläche (13) eine Ausnehmung (13') vorhanden ist zur Aufnahme eines Endes der Saugleitung (5) und dass mindestens ein Bedienungsmittel (14) zur Betätigung der Saugpumpe vorhanden ist und dass das mindestens eine Bedienungsmittel (14) Bedienungstasten (15) sind, welche in der Vorderseite (11) des Gehäuses (10) eingelassen sind.

2. Saugeinheit (1) zur Verwendung in einer portablen Brustpumpe gemäss Anspruch 1, wobei die Brustpumpe eine Auffangeinheit (A) mit einer Brustaufsatzhaube (2), einem Milchaufnahmebehälter (4) und einem die Brustaufsatzhaube (2) mit dem Milchaufnahmebehälter (4) verbindenden Kopplungselement (3) aufweist, wobei die Brustpumpe ferner eine flexible Saugleitung (5) aufweist, mittels welcher die Auffangeinheit (A) mit der Saugeinheit (1) verbindbar ist, und wobei die Saugeinheit (1) ein Gehäuse (10) aufweist, wobei das Gehäuse (10) mit einem Befestigungsmittel (15) versehen ist zur Befestigung des Gehäuses (10) an einem Tragmittel (G,S), welches am Körper einer Mutter befestigbar ist, wobei das Gehäuse (10) eine Vorderseite (11), eine Rückseite (12) sowie eine die zwei Seiten (11, 12) verbindende Mantelfläche (13) aufweist und wobei das Gehäuse (10) flach ausgebildet ist, in dem Sinne, dass die Vorderseite (11) und die Rückseite (12) breiter ausgebildet sind als die Mantelfläche (13), **dadurch gekennzeichnet, dass** in der Mantelfläche (13) eine Ausnehmung (13') vorhanden ist zur Aufnahme eines Endes der Saugleitung (5) und dass mindestens ein Bedienungsmittel (14) zur Betätigung der Saugpumpe vorhanden ist und dass das mindestens eine Bedienungsmittel (14) Bedienungstasten (15) sind, welche in der Vorderseite (11) des Gehäuses (10) eingelassen sind.

3. Saugeinheit nach Anspruch 2, **dadurch gekennzeichnet, dass** das Befestigungsmittel (15) zur Befestigung an einem Gürtel (G) oder an einer Tragschlaufe (S) ausgebildet ist.

4. Saugeinheit nach einem der Ansprüche 2 oder 3, **dadurch gekennzeichnet, dass** das Befestigungsmittel (15) als lösbares Mittel ausgebildet ist.

5. Saugeinheit nach einem der Ansprüche 2 bis 4, **dadurch gekennzeichnet, dass** das Gehäuse (10) eine Rückseite (12) aufweist und dass das Befestigungsmittel (15) an dieser Rückseite (12) angeordnet ist.

6. Saugeinheit nach einem der Ansprüche 2 bis 5, **dadurch gekennzeichnet, dass** das Befestigungsmittel (15) eine gefederte Klammer (15') und/oder ein Steckverschlussteil (15") ist.

7. Saugeinheit nach einem der Ansprüche 2 bis 6, **dadurch gekennzeichnet, dass** das Gehäuse (10) starr ausgebildet ist.

## Claims

1. A portable breast pump which comprises a suction unit (1), a collecting unit (A) and a suction line (5), the suction line (5) connecting the collecting unit (A) to the suction unit (1), the collecting unit (A) having a breast shield (2), a milk-receiving container (4) and a coupling element (3), which connects the breast shield (2) to the milk-receiving container (4), and the suction unit (1) having a housing (10), wherein the housing (10) is provided with a fastening means (15) for fastening the housing (10) on a carrying means (G, S), which can be fastened on a mother's body, wherein the housing (10) comprises a front side (11), a rear side (12) as well as a lateral surface (13) which connects the two sides (11, 12) and wherein the housing (10) is of flat design, in the sense, that the front side (11) and the rear side (12) are of broader design than the lateral surface (13), **characterized in that** a recess (13') is provided in the lateral surface (13) for the purpose of accommodating one end of the suction line (5) and **in that** at least one operating means (14) for operating the suction pump is provided and **in that** the at least one operating means (14) are operating buttons, which are embedded in the front side (11) of the housing (10).

2. A suction unit (1) for use in a portable breast pump as claimed in claim 1, the breast pump having a collecting unit (A) with a breast shield (2), a milk-receiving container (4) and a coupling element (3), which connects the breast shield (2) to the milk-receiving container (4), the breast pump also having a flexible suction line (5), by means of which the collecting unit (A) can be connected to the suction unit (1), and the suction unit (1) having a housing (10), wherein the housing (10) is provided with a fastening means (15) for fastening the housing (10) on a carrying means (G, S), which can be fastened on a mother's body, wherein the housing (10) comprises a front side (11), a rear side (12) as well as a lateral surface (13) which connects the two sides (11, 12) and wherein the housing (10) is of flat design, in the sense, that the front side (11) and the rear side (12) are of broader design than the lateral surface (13), **characterized in that** a recess (13') is provided in the lateral surface (13) for the purpose of accommodating one end of the suction line (5) and **in that** at least one operating means (14) for operating the suction pump is provided and **in that** the at least one operating means (14) are operating buttons, which are embedded in the front side (11) of the housing (10).

3. The suction unit according to claim 2, **characterized in that** the fastening means (15) is designed for fastening on a belt (G) or on a carrying loop (S).

4. The suction unit according to claim 2 or 3, **characterized in that** the fastening means (15) is designed as a releasable means.

5. The suction unit according to one of claims 2 to 4, **characterized in that** the housing (10) comprises a rear side (12) and **in that** the fastening means (15) is arranged on that rear side (12).

6. The suction unit according to one of claims 2 to 5, **characterized in that** the fastening means (15) is a resilient clip (15') and/or a plug-in closure part (15").

7. The suction unit according to one of claims 2 to 6, **characterized in that** the housing (10) is of rigid design.

## Revendications

1. Tire-lait portatif, présentant une unité de succion (1), une unité de collecte (A) et une conduite de succion (5), la conduite de succion (5) reliant l'unité de collecte (A) à l'unité de succion (1), l'unité de collecte (A) présentant une téterelle (2), un récipient de collecte du lait (4) et un élément d'accouplement (3) reliant la téterelle (2) au récipient de collecte du lait (4) et l'unité de succion (1) présentant un boîtier (10), le boîtier (10) étant muni d'un moyen de fixation (15) pour la fixation du boîtier (10) sur un moyen de support (G, S), qui peut être fixé au corps d'une mère, le boîtier (10) présentant un côté avant (11), un côté arrière (12) ainsi qu'une surface d'enveloppe (13) reliant les deux côtés (11, 12) et le boîtier (10) étant réalisé sous forme plate en ce sens que le côté avant (11) et le côté arrière (12) sont réalisés plus larges que la surface d'enveloppe (13), **caractérisé en ce que** dans la surface d'enveloppe (13) est prévu un évidement (13') pour recevoir une extrémité de la conduite de succion (5) et **en ce qu'**au moins un moyen de commande (14) est prévu pour l'actionnement du tire-lait et **en ce que** l'au moins un moyen de commande (14) est constitué par des touches de commande qui sont incorporées dans le côté avant (11) du boîtier (10).

2. Unité de succion (1) pour l'utilisation dans un tire-lait portatif selon la revendication 1, dans laquelle le tire-lait présente une unité de collecte (A) avec une téterelle (2), un récipient de collecte du lait (4) et un élément d'accouplement (3) reliant la téterelle (2) au récipient de collecte du lait (4), le tire-lait présentant en outre une conduite de succion flexible (5) au moyen de laquelle l'unité de collecte (A) peut être connectée à l'unité de succion (1), et l'unité de succion (1) présentant un boîtier (10), le boîtier (10) étant muni d'un moyen de fixation (15) pour la fixation du boîtier (10) sur un moyen de support (G, S) qui peut être fixé au corps d'une mère, le boîtier (10) présentant un côté avant (11), un côté arrière (12) ainsi qu'une surface d'enveloppe (13) reliant les deux côtés (11, 12) et le boîtier (10) étant réalisé sous forme plate en ce sens que le côté avant (11) et le côté arrière (12) sont réalisés plus larges que la surface d'enveloppe (13), **caractérisée en ce que** dans la surface d'enveloppe (13) est prévu un évidement (13') pour recevoir une extrémité de la conduite de succion (5) et **en ce qu'**au moins un moyen de commande (14) est prévu pour l'actionnement du tire-lait et **en ce que** l'au moins un moyen de commande (14) est constitué par des touches de commande qui sont incorporées dans le côté avant (11) du boîtier (10).

3. L'unité de succion selon la revendication 2, **caractérisée en ce que** le moyen de fixation (15) est réalisé pour la fixation sur une ceinture (G) ou une lanière en boucle (S).

4. L'unité de succion selon l'une quelconque des revendications 2 ou 3, **caractérisée en ce que** le moyen de fixation (15) est réalisé sous forme de moyen amovible.

5. L'unité de succion selon l'une quelconque des revendications 2 à 4, **caractérisée en ce que** le boîtier (10) présente un côté arrière (12) et **en ce que** le moyen de fixation (15) est disposé sur ce côté arrière (12).

6. L'unité de succion selon l'une quelconque des revendications 2 à 5, **caractérisée en ce que** le moyen de fixation (15) est une pince à ressort (15') et/ou une pièce de fermeture par enfichage (15").

7. L'unité de succion selon l'une quelconque des revendications 2 à 6, **caractérisée en ce que** le boîtier (10) est réalisé sous forme rigide.
